# EUROPEAN PATENT SPECIFICATION

(11) **EP 1 557 456 B1**
(45) Date of publication and mention of the grant of the patent: **13.01.2016**
(21) Application number: 03809845.5
(22) Date of filing: 16.09.2003
(51) Int. Cl.: C09K 21/08, C08K 5/00, C08L 101/00, C07C 23/12, C07C 23/28, C08K 5/02

(54) **FLAME RETARDANT FOR PLASTIC**
FLAMMSCHUTZMITTEL FüR KUNSTSTOFF
IGNIFUGE POUR PLASTIQUE

(30) Priority: 29.10.2002 JP 2002313690
(43) Date of publication of application: 27.07.2005
(73) Proprietor: Dai-Ichi Kogyo Seiyaku Co., Ltd., Kyoto-shi, Kyoto 600-8873 (JP)
(72) Inventor: ONISHI, Hideaki, Otsu-shi, Shiga 520-0821 (JP); TERAMOTO, Makoto, Kyoto-shi, Kyoto 615-0064 (JP)
(74) Representative: Reitstötter Kinzebach
(86) International application number: PCT/JP2003/011786
(87) International publication number: WO 2004/039917

(56) References cited:
- EP-A1- 0 027 556
- EP-A1- 0 117 186
- WO-A1-2004/016712
- DE-A1- 2 820 410
- DE-A1- 10 123 584
- GB-A- 1 376 822
- GB-A- 1 396 657
- GB-A- 2 308 600
- JP-A- 55 157 519
- JP-A- 60 181 037
- JP-A- 61 223 055
- US-A- 3 919 356
- US-A- 3 932 543
- US-A- 4 419 458

## Description

### [Field of The Invention]

This invention relates to a flame retarded plastic composition.

### [Background Art]

A wide variety of plastic materials are being used in many fields such as electric and electronic equipments, automotives, building and construction materials. Styrene- and olefin-based resins are the largest in use among others by virtue of their versatility and cost-effectiveness. Inflammable plastic materials are required to be flame-retarded in many application fields to fulfill respective flame retardancy standard.

A variety of flame retardants may be used for styrene- and olefin-based plastics. Among them, halogen-containing, typically bromine-containing organic compounds are effective with minimum deteriorateive effects on physical properties of resins. The bromine-containing organic compounds may be classified into of aromatic type in which bromine are attached to aromatic carbon atoms and of aliphatic type in which bromine are attached to aliphatic or cycloaliphatic carbon atoms. It is said that bromine atoms attached to an alkyl or cycloalkyl group are generally superior in flame retardancy compared to bromine atoms attached to aromatic ring carbon atoms. Representative examples of aliphatic brominated compounds includes hexabromocyclododecane (HBCD) and tetrabromocyclooctane (TBCO). They have long been used in building and construction materials, electrical equipments. HBCD has been most commonly used for building and construction materials made of foamed polystyrene as described in JP-2002-194130A and JP 2002-30174A. JP 07179647A discloses use of TBCO in polystyrene foams. However, HBCD is not satisfactory compared to TBCO in flame retardancy while TBCO generates a unique, unpleasant odor. US 4,419,458 and GB 1376822 disclose flame retardant compositions comprising styrene polymers and organic halogen compounds as flame retardant agents. The organic halogen compounds are bromine compounds, such as brominated oligomers of butadiene or isoprene. EP 0 027 556 A1 discloses flame retarded foamed styrene polymers which may contain organic bromo compounds as flame retardant agents. US 3,932,543 discloses halogenated isoprene derivatives, such as myrcene, ocimene, or terpinene, which are useful as perfumes and flame retardants. US 3,919,356 discloses flame retardant polymeric compositions comprising halogenated Diels-Alder products. JP 55 157519 A discloses a process for the selective dibromination of the terminal double bond of a terpene compound using TBCO. JP 60 181037 A discloses a process for the bromination of cyclic compounds using TBCO.

It is, therefore, an object of the present invention to provide a flame-retardant composition for plastic materials in which all bromine atoms are attached to an aliphatic or cycloaliphatic hydrocarbon reside and thus exhibiting enhanced flame retarding effects without generating unpleasant odors.

### [Disclosure of The Invention]

The present invention provides a flame retarded plastic composition comprising a styrene-based plastic material and 1 to 5 parts by weight per 100 parts by weight of said material of an addition reaction product of bromine and a mono⁻ or sesquiterpene compound selected from the group consisting of myrcene, ocimene, limonene, terpinolene, sylvestrene, carene, bisabolene, farnesene, curcumene, cadinene and sesquibenihene. The term "terpene" refers in the broadest sense to a series of terpene hydrocarbons of the formula (C₅H₈)n but also oxygen-containing derivative thereof such as alcohols, aldehydes and ketones. Terpenes of the above formula are called "monoterpenes" when n is 2, "sesquiterpenes" when n is 3, and "diterpenes" when n is 4. The term "terpene compound" as used herein refers to "terpenoid". In order to produce a flame retardant through bromination, monoterpenes such as myrcene, ocimene, limonene, terpinolene, sylvestrene, or carene; and sesquiterpenes such as bisabolene, farnesene, curcumene, cadinene, or sesquibenihene are employed as starting terpene compounds.

Terpene compounds have at least one unsaturated double bond to which two bromine atoms may be addition reacted. The addition reaction of bromine may be accomplished by the method known per se. For example, an amount of bromine is dissolved in an alcoholic solvent such as isobutanol or a halogenated hydrocarbon solvent such as dichloromethane, chloroform or carbon tetrachloride. To the bromine solution is added dropwise a theoretical amount of a starting terpene compound with stirring. Alternatively, Bromine and the starting terpene compound each in a theoretical amount are concurrently added dropwise to the above solvent with stirring. Preferably the reaction is carried out at room temperature or a temperature below the room temperature. After the reaction, the reaction mixture is neutralized if necessary and filtered to recover the reaction product which is then washed with water and dried to obtain the desired addition reaction product of bromine and a mono- or sesquiterpene. The resulting brominated terpene compound is essentially comparable to TBCO in flame retardancy but does not generate unpleasant odor.

The quantity of the addition reaction product of bromine and a mono⁻ or sesquiterpene compound selected from the group consisting of myrcene, ocimene, limonene, terpinolene, sylvestrene, carene, bisabolene, farnesene, curcumene, cadinene and sesquibenihene may vary depending on the bromine content of a particular compound, the required level of flame retardancy, and co-addition of auxiliary flame retardants such as antimony trioxide, and generally ranges from 1 to 5 parts by weight per 100 parts by weight of the plastic material.

### [Examples]

The invention is further illustrated by the following Examples and Comparative Examples which are not intended to limit the invention thereto. In Examples, the bromine content was determined by a method similar to the method according to JIS K 72 29 (quantitative analysis of chlorine in chlorine-containing resins). Melting points were determined using a melting point tester (FP90/HT sold by Mettler Toledo Co., Ltd.) at a temperature elevating rate of 3 °C /min.

### Example 1

A 100ml four necked flask equipped with a thermometer, two drip funnels and a stirrer was charged with 35g of isobutanol. To the flask were added dropwise 13.1g of limonene and 32.1g of bromine concurrently while stirring at a temperature below 20 °C. After the addition, the mixture was allowed to react for one hour at a temperature of 20-30 °C. After the reaction, the reaction mixture was neutralized to pH 7-8 with monoethanolamine, filtered to recover solids. A brominates limonene compound hereinafter called "compound B-1" having a bromine content of 70.6% and a melting point of 112°C was obtained by washing the solids with water and then drying.

### Comparative Example 1

A 100ml four necked flask equipped with a thermometer, two drip funnels and a stirrer was charged with 35g of isobutanol. To the flask were added dropwise 13.6g of phellandrene and 32.1g of bromine concurrently while stirring at a temperature below 20 ° C. After the addition, the mixture was allowed to react at a temperature of 20-30 °C, neutralized to pH 7-8 with monoethanolamine and then filtered to recover solids. A brominated phellandrene compound hereinafter called "compound B-2" having a bromine content of 70.8% and a melting point of 92 °C was obtained by washing the solids with water and then drying.

### Example 2

A 100ml four necked flask equipped with a thermometer, two drip funnels and a stirrer was charged with 35g of isobutanol. To the flask were added dropwise 14.3g of bisabolene and 32.1g of bromine concurrently while stirring at a temperature below 20 °C. After the addition, the mixture was allowed to react at 20-30 °C for one hour, neutralized to pH 7-8 with monoethanolamine and then evaporated to remove the solvent. The residue was re-dissolved in 100ml of methylene chloride, washed with water and then evaporated to remove the solvent whereupon a brominated bisabolene compound herein after called "compound B-3" was obtained as a viscous yellow liquid having a bromine content of 52.3%.

### Example 3

A 100ml four necked flask equipped with a thermometer, two drip funnels and a stirrer was charged with 35g of isobutanol. To the flask were added dropwise 14.3g of farnesene and 46.3g of bromine concurrently while stirring at a temperature below 20 °C . After the addition, the mixture was allowed to react at 20-30 °C for one hour, neutralized to pH 7-8 with monoethanolamine and then evaporated to remove the solvent. The residue was re-dissolved in 100ml of methylene chloride, washed with water and then evaporated to remove the solvent whereupon a brominated farnesene compound hereinafter called "compound B-4" was obtained as a yellow viscous liquid having a bromine content of 64.1%.

### Examples 4-6 and Comparative Examples 2-7

Polystyrene resin was flame-retarded using brominated terepenes synthesized in the preceding Examples and known flame-retardants. The resulting compositions were tested for flame retardancy and odor.

The materials used are as follows.

### A. Polystyrene

TOYO STYROL G220 available from Toyo Styrene Co., Ltd.

### B. Flame retardants

B-1: Compound B-1 produced in Example 1
B-2: Compound B-2 produced in Comparative Example 1
B-3: Compound B-3 produced in Example 2
B-4: Compound B-4 produced in Example 3
B-5: Hexabromocyclododecane (HBCD)
B-6: Tetrabromocyclooctane (TBCO)
B-7: 2,2-Bis[4-(2,3-dibromopropoxy)-3,5-dibromophenyl]propane, available under the name of PYROGUARD SR-720 from Dai-Ichi Kogyo Seiyaku, Co., Ltd.
B-8: Bis(pentabromophenyl)ethane, available under the name of SAYTEX 8010 from Albemarle Corp.
B-9: Brominated epoxy resin oligomer available under the name of YDB-409 from Tohto Kasei Co., Ltd.

### C. Other additives

C-1: Heat stabilizer available under the name of STANN BM(N) from Sankyo Organic Chemicals Co., Ltd.
C-2: Antioxidant available under the name of ADK STAB PEP-36 available from Asaki Denka Kogyo Co., Ltd.

### Preparation of specimens for evaluation of flame retardancy

To 100 parts by weight of polystyrene were mixed 3 parts of weight of the flame retardant, 0.05 parts by weight of additive C-1 and 0.01 parts by weight of additive C-2. The mixture was kneaded in a hot roll mill at 200 °C for five minutes and then compressed into a sheet in a hot press at 200 °C for 3 minutes. Specimens were cut from the sheet.

### Burning test:

The oxygen index (LOI) of each specimen was determined according to the method of JIS K 7201.

### Odor test

Each of flame retardants B-1 to B-9 was separately taken in a 10ml sample bottle in an amount of 10g/bottle. The odor of each compound was evaluated by 20 panelists. In this test, the odor was judged according to the following schedule.
Bad: more than 15 panelists reported to be unpleasant.
Fair: 6-15 panelists reported to be unpleasant
Good: 0-5 panelists reported to be unpleasant.

The results are shown in the table below.

| | Example | | | Comparative Example | | | | | |
|---|---|---|---|---|---|---|---|---|---|
| | 4 | 5 | 6 | 2 | 3 | 4 | 5 | 6 | 7 |
| Flame retardant | B-1 | B-3 | B-4 | B-2 | B-5 | B-6 | B-7 | B-8 | B-9 |
| Burning test (LOI) | 26.9 | 25.3 | 25.8 | 26.6 | 23.0 | 25.8 | 22.3 | 19.6 | 18.4 |
| Odor test | Good | Good | Good | Good | Fair | Bad | Good | Good | Good |

As shown in the above table, Compounds B1, B3 and B4 and Comparative Compound B2 exhibited enhanced flame retarding effects without generating unpleasant odor. Among brominate compounds in Comparative Examples 3 to 7, compound B-6 was satisfactory in the flame retarding effect but generated unpleasant odor. All other compounds, compounds B-5 and B-7 to B-9 were not satisfactory in the flame retarding effect.

## Claims

1. A flame retarded plastic composition comprising a styrene-based plastic material and 1 to 5 parts by weight per 100 parts by weight of said material of an addition reaction product of bromine and a mono- or sesquiterpene compound selected from the group consisting of myrcerie, ocimene, limonene, terpinolene, sylvestrene, carene, bisabolene, farnesene, curcumene, cadinene and sesquibenihene.

2. A flame retarded plastic composition according to claim 1 wherein said mono- or sesquiterpene compound is limonene, bisabolene or farnesene.

## Patentansprüche

1. Flammhemmende Kunststoffzusammensetzung, umfassend ein Styrol-basiertes Kunststoffmaterial und 1 bis 5 Gewichtsteile eines Additionsreaktionsprodukts von Brom und einer Mono- oder Sesquiterpenverbindung pro 100 Gewichtsteile des Materials, wobei die Mono- oder Sesquiterpenverbindung unter Myrcen, Ocimen, Limonen, Terpinolen, Sylvestren, Caren, Bisabolen, Farnesen, Curcumen, Cadinen und Sesquibenihen ausgewählt ist.

2. Flammhemmende Kunststoffzusammensetzung nach Anspruch 1, wobei die Mono- oder Sesquiterpenverbindung Limonen, Bisabolen oder Farnesen ist.

## Revendications

1. Composition plastique ignifugée comprenant une matière plastique à base de styrène et 1 à 5 parts en poids pour 100 parts de poids de ladite matière d'un produit de réaction d'addition du brome sur un composé mono- ou sesquiterpène choisi parmi le groupe formé par le myrcène, l'ocimène, le limonène, le terpinolène, le sylvestrène, le carène, le bisabolène, le farnésène, le curcumène, le cadinène et le sesquibénihène.

2. Composition plastique ignifugée selon la revendication 1 dans laquelle ledit composé mono- ou sesquiterpène est le limonène, le bisabolène ou le farnésène.
